# EUROPEAN PATENT APPLICATION

(11) **EP 1 350 474 A1**
(43) Date of publication of application: **08.10.2003**
(21) Application number: 03251418.4
(22) Date of filing: 07.03.2003
(51) Int. Cl.: A61B 17/24

(54) **Scraper**

(30) Priority: 19.03.2002 GB 0206397
(71) Applicant: Peri-dent Limited, Galashiels, Selkirkshire TD1 3RS, Scotland (GB)
(72) Inventor: Barlow, David, c/o Peri-dent Limited, Galashiels, Selkirkshire TD1 3RS (GB); Hill, James McKenzie, c/o Peri-dent Limited, Galashiels, Selkirkshire TD1 3RS (GB)
(74) Representative: Parnham, Kevin

(57) **Abstract**

A scraper 1, 11, 21, 31, 41, 51, 61 is provided which may be of a disposable nature. The scraper 1, 11, 21, 31, 41, 51, 61 generally has an arcuate head 2, 12, 22, 32, 42, 52, 62 which presents a distal end to compliantly engage a user's tongue. A distal end is typically formed from an elastomeric material in order to sympathetically engage the tongue whilst scraping but resistant a natural gagging reflex inherent with such scraping. A number of alternative embodiments of the scraper are provided in order to allow different degrees of construction complexity, cost and efficiency of performance.

## Description

The present invention relates to a scraper or cleaner and more particularly to a scraper used with regard to hygienically scraping or cleaning a human tongue.

Recent research has indicated that a significant cause of halitosis is bacteria deposited towards the rear of a human tongue. Typically, front and side portions of a human tongue are naturally scraped by other parts of the mouth during chewing and throughout the day whilst the base or back of the tongue is not cleaned such that bacteria can build up there. In such circumstances, there is a desire to remove bacteria from the back of the tongue in order to achieve more hygienic and fresher breath. Unfortunately, there is a natural "gagging" reflex whenever the rear or back of a mouth is depressed or contacted and so use of a toothbrush is unacceptable.

Tongue scrapers have been developed which are typically of a limited life or usage. These tongue scrapers normally comprise a long handle and a round cleaning head which may be made from a plastics material or stainless steel or copper. It will be appreciated that in order to provide the necessary scraping motion the tongue scraper must be sufficiently rigid to allow judicious application of scraping pressure. Thus, present tongue scrapers typically include relatively robust strips of plastics material or metals such as copper or stainless steel in order to allow scraping. Limitations with regard to the materials from which the scraper can be made limit the acceptability of providing a scraper as a disposable item.

In accordance with the present invention there is provided a scraper for a human tongue, the scraper comprising an arcuate head with a distal end formed to compliantly engage upon a tongue.

Preferably, the arcuate head is oval or round or an arch for structural stability to facilitate compliant engagement on a tongue.

Preferably, the scraper includes a handle upon which the arcuate head is secured. Typically, the arcuate head is integral with the scraper. Possibly, the arcuate head is detachable from the scraper. Typically, the arcuate head is held in keyed engagement with the scraper. Normally, the arcuate head, when detachable from the scraper, will be locked in place during use.

Preferably, the distal end has a scraper edge for sympathetic engagement with a tongue. Normally, the scraper edge will be made from an elastomeric material. Possibly, the distal end will be serrated or otherwise shaped to facilitate scraping of a tongue. Advantageously, the distal end or the scraper edge is detachable from the arcuate head. Typically, the distal end will have a bow or bite for progressive engagement with a tongue as the scraper is drawn across a tongue.

Preferably, the scraper is formed from a plastics material which is conveniently cleaned. Alternatively, the arcuate head is detachable for cleaning. Possibly, the distal end or the scraper edge includes an anti-bacterial agent in its material or may be charged with an anti-bacterial agent or breath freshener. Advantageously, at least the distal end or the scraper edge will include an agent to indicate either that the scraper should be cleaned or the anti-bacterial agent is exhausted or that the scraper should be replaced.

Embodiments of the present invention will now be described by way of example with reference to the accompanying drawings in which:
Fig. 1 is a plan view of a first embodiment of a scraper;
Fig. 2 is an exploded perspective view of the scraper depicted in Fig. 1;
Fig. 3 is a plan view of a second embodiment of a scraper;
Fig. 4 is an exploded perspective view of the scraper depicted in Fig. 3;
Fig. 5 is a plan view of a third embodiment of a scraper;
Fig. 6 is an exploded perspective view of the scraper depicted in Fig. 5;
Fig. 7 is a plan view of a fourth embodiment of a scraper;
Fig. 8 is an exploded perspective view of the scraper depicted in Fig. 7;
Fig. 9 is a plan view of a fifth embodiment of a scraper;
Fig. 10 is an exploded perspective view of the scraper depicted in Fig. 9
Fig. 11 is a plan view of a sixth embodiment of a scraper;
Fig. 12 is a perspective view of the scraper depicted in Fig. 11 with its scraper edge detached;
Fig. 13 is a plan view of a seventh embodiment of a scraper; and,
Fig. 14 is an exploded perspective view of the scraper depicted in Fig. 13.

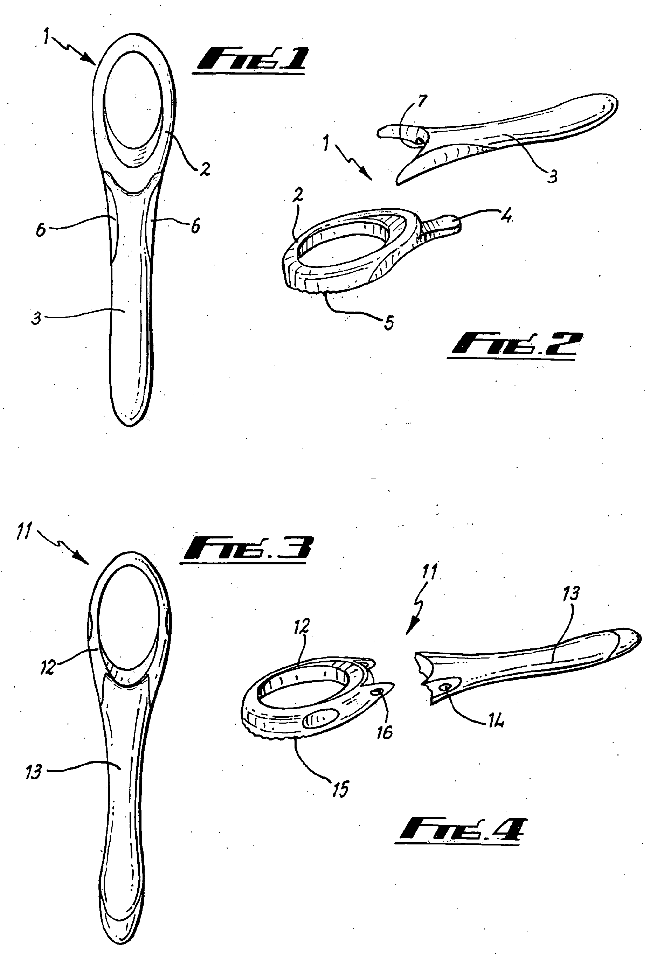

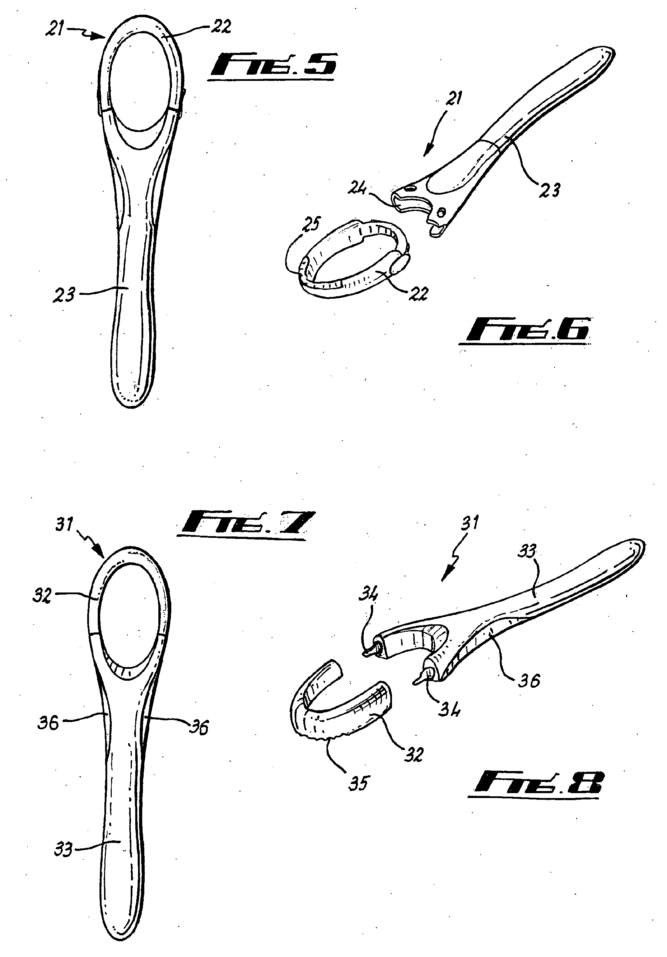

Referring to Figs. 1 and 2 it will be seen that a first embodiment of a scraper 1 in accordance with the present invention comprises an arcuate head 2 secured to a handle 3 with a keyed coupling 4. The arcuate head 2 includes a scraper edge 5 which in use is generally compliant in engagement with a human tongue. The scraper edge 5 will normally be formed with an elastomeric nature in order to sympathetically engage a tongue and so resist the natural "gagging reflex" as the rear of a human tongue is compressed and contacted.

The arcuate member 2 is formed as a general oval in order to provide structural stability for controlled and substantially rigid presentation of the scraper 2 by a user upon their tongue. It will be understood that if the scraper 2 is overly flexible it may be difficult to accurately place the scraper upon the tongue and apply the desired slight pressure to scrape. An arcuate form with an oval or round or at least an arched distal end including the scraper edge 5 readily achieves the necessary component predictability without requiring thick material cross-sections or use of metal components.

Typically, the handle 3 incorporates grip sides 6 to again facilitate accurate user control and positioning of the scraper 2 in use. Advantageously these gripper portions 6 are coloured so that a user is drawn to correctly gripping the scraper 2 by engaging these gripper sections 6 between a finger and thumb with the remainder of the handle 3 cupped in the palm of a user's hand.

The keyed coupling 4 engages a reciprocal recess or cut-out in the handle 3 such that there is secure location between the head 2 and the handle 3 in use. In such circumstances, the head 2 can be removed for cleaning or replacement when worn out or otherwise unacceptable for further operation. Furthermore, the keyed coupling 4 in engagement with the recess 7 may allow a degree of up/down pivot for more appropriate presentation of the head and in particular the scraper edge 5 to a user's tongue.

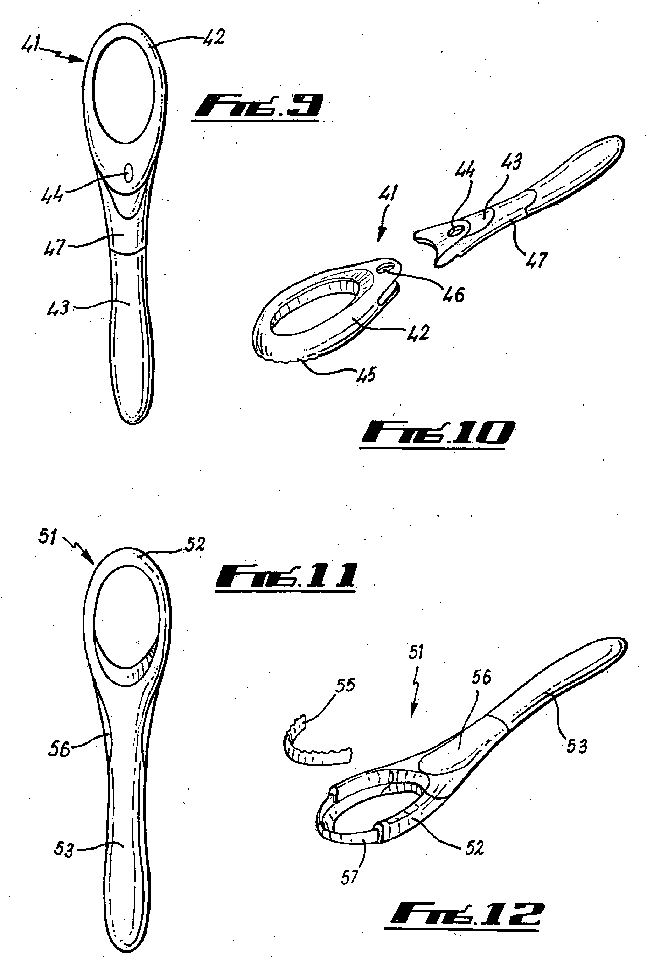

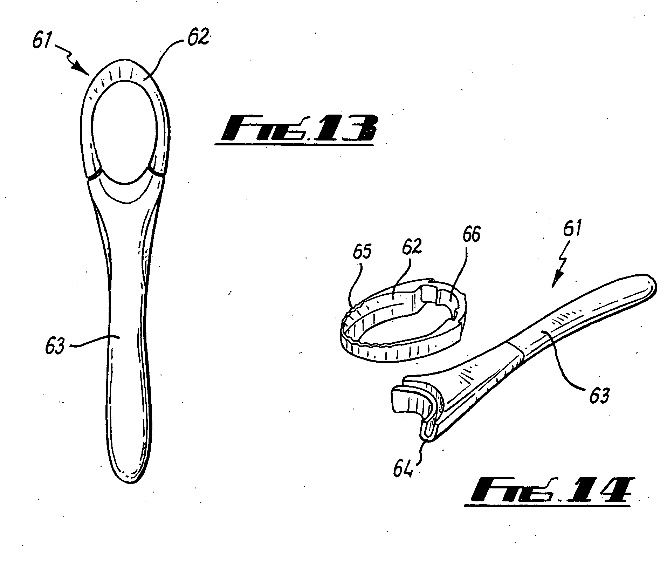

Referring to Figs. 3 and 4 illustrating a second embodiment of a scraper 11 in accordance with the present invention. Thus, the scraper 11 comprises an arcuate head 12 and a handle 13 secured together in order to allow appropriate presentation of the scraper 11 to a user's tongue. The arcuate head 12 incorporates a scraper edge 15 and is secured to the handle 13 through opposed posts 14 in engagement with hinge apertures 16 such that in use a degree of up/down motion about the hinge formed between posts 14 and apertures 16 is allowed for acceptable presentation of the scraper and in particular the scraper edge 15 to a user's tongue.

The arcuate head 12 is again substantially oval in shape in order to provide relative stability in the plane of the head 12 for consistent and predictable presentation of the scraper edge 15 in use.

With scraper 11, the whole of the handle 13 is covered or formed from an appropriate material for easy gripping by a user in their most desired manner.

The head 12 is detachable by use of excessive force to release the apertures 16 from the posts 14. Thus, the head 12 can be replaced or cleaned as required.

Referring to Figs. 5 and 6 showing a third embodiment of a scraper 21 in accordance with the present invention. Thus, an arcuate head 22 is secured to a handle 23 through a lock mechanism comprising an entrant slot 24 which encapsulates a near end of the arcuate head 22 and secures location through detent engagement between respective latch elements in the head 22 and the handle 23.

The arcuate head 22 essentially comprises an oval hoop of normally plastics material. Being oval in cross-section it will be appreciated that the arcuate head 22 is relatively structurally stable and so consistently presents a scraper edge 25 to a user's tongue in use. The arcuate head 22 is fixed through the entrant engagement and detent locking with the handle 23 such that no flexibility through a hinge mounting between the head 22 and the handle 23 is possible. Thus, only a lower degree of flexibility will be possible through the nature of the plastics materials from which the head 22 and handle 23 are formed. The scraper 21 will be a substantially stable longitudinal assembly.

The scraper edge 25 is located upon the distal end of the head 22. The scraper edge 25 is formed from an elastomeric insert appropriately secured to the head 22. Typically, the scraper edge 25 will be serrated or otherwise shaped for sympathetic engagement with a user's tongue. Furthermore, the scraper edge 25 will normally be formed from a synthetic rubber appropriately shaped and moulded for engagement upon a rib mounting formed in the head 22. In such circumstances, the scraper edge 25 may be removed and replaced or cleaned as required.

Referring to Figs. 7 and 8 showing a fourth embodiment of a scraper 31 in accordance with the present invention. Thus, an arcuate head 32 is secured to a handle 33 through mounting spigots 34. The arcuate head 32 includes a scraper edge 35 for compliant and sympathetic engagement with a user's tongue in use.

The arcuate head 32 is formed by a U-shaped bow engaging the spigots 34 in order to create the structurally stable oval or arcuate cross-section for consistent presentation of the scraper edge 35 to a user's tongue. Other than any inherent flexibility in the materials from which the scraper 31 is formed, it will be appreciated that there is no flexibility in the scraper 31 and so there is consistent presentation of the scraper edge 35 to a user's tongue whilst enabling that scraper 31 to be formed from relatively light plastics material. The handle 33 includes grip sections 36 to enable a user to appropriately position and locate the scraper 31 relative to their tongue.

In use, the U-shaped bow is detachable from the spigots 34 for cleaning or to enable replacement in the scraper 31. The U-shaped bow will be formed from a relatively flexible material in order to ensure that the scraper edge 35 is compliant and possibly has an elastomeric nature for appropriate sympathetic engagement with a user's tongue. In such circumstances, the handle 33 can be relatively rigid for appropriate presentation of the scraper 31 whilst only the U-shaped bow of the arcuate head 36 is formed from softer material for compliant engagement with a user's tongue.

Referring to Figs. 9 and 10 which show a fifth embodiment of a scraper 41 in accordance with the present invention. Thus, an arcuate head 42 is secured to a handle 43 through a radial hinge mounting 44 which comprises a hinge pin engaging a hinge aperture 46 to allow side-to-side motion of the arcuate head 42 in use. The arcuate head 42 includes a scraper edge 45 for sympathetic engagement with a user's tongue in use. Furthermore, the arcuate head 42 again has a substantially oval cross-section for consistent presentation of the scraper edge 45 whilst the flexibility allowed by the hinge mounting 44 allows adjustment of the angle of presentation of the scraper edge 44 by the user. It will be appreciated that the scraper 44 could be configured such that the head 42 can pivot about the hinge 44 as the scraper 41 is drawn across a user's tongue or alternatively the angle of the head 42 relative to the handle 43 could be set prior to use and in order to allow better presentation to one side of the rear of a user's tongue.

The handle 43 includes a gripper section 47 which will generally be pinched between a user's finger and thumb with the remainder of the handle 43 cupped by the palm of a user's hand in order to facilitate appropriate presentation of the scraper edge 45 to a user's tongue.

Referring to Figs. 11 and 12 illustrating a sixth embodiment of a scraper 51 in accordance with the present invention. Thus, an arcuate head 52 is integrally formed with a handle 53 such that a scraper edge 55 is appropriately presented to a user's tongue in use. Typically, apart from the scraper edge 55, the arcuate head 52 and the handle 53 will be a stable combination including a gripper section 56 to be engaged by a user for appropriate presentation of the scraper 51 in use. Thus, other than the inherent flexibility in the materials from which the scraper 51 is formed there is no flexibility in the scraper 51. In such circumstances, the arcuate head 52 through its structural configuration has an oval cross-section to robustly present the scraper edge 55 in a stable manner despite being formed from relatively thin cross-section and flexible plastics materials.

The scraper edge 55 will normally be formed from a synthetic rubber material in order to be elastomeric for sympathetic and compliant presentation with a user's tongue. The scraper edge 55 is secured upon a rail section 57 and is detachable therefrom for replacement or cleaning. The scraper edge 55 is typically serrated or otherwise shaped for appropriate presentation and engagement with a user's tongue.

Referring to Figs. 13 and 14 showing a seventh embodiment of a scraper 61 in accordance with the present invention. Thus, an arcuate head 62 is secured to a handle 63 through an interference engagement between a channel 64 and a gripper end 66 of the arcuate head 62. The arcuate head 62 includes at a distal end a scraper edge 65 which is arranged to sympathetically engage a user's tongue in use.

The whole of the handle 62 will be relatively rigid for appropriate manipulation and presentation of the arcuate head 62. The arcuate head 62 has a substantially oval cross-section for structural rigidity relative to the materials and cross-section of the arcuate head. Thus, the scraper edge 65 will be predictably presented by the scraper 61 to enable better positioning by a user in sympathetic engagement in use with a user's tongue.

The arcuate head 62 at the distal end including the scraper edge 65 has a relatively thin cross-section which expands into a more substantial cross-section nearer to the gripper end 62. Thus, in combination with the handle 63 about the channel 64, the whole combination is relatively rigid in order to achieve the desired consistent presentation of the scraper edge 65. By such means, the material from which the arcuate head 62 as a closed loop is formed can be a relatively flexible material such that the distal end including the scraper edge 65 is then compliant and typically has an elastomeric nature for the desired sympathetic engagement with a user's tongue.

In the present invention it is use of the arcuate head in the form of an oval or round or arch cross-section which provides relative structural stability in comparison with the materials from which the arcuate head is formed. With relative structural stability it will be appreciated that the scraper edge can be more accurately presented for slight compressive engagement in order to clean a user's tongue by scraping that edge across the rear of the tongue to remove debris and bacteria. Furthermore, the distal end of the arcuate head will generally include a bow or bite configuration such that there is progressive engagement with the tongue as the scraper is drawn across it. Initially, a narrow front part of the scraper edge engages the tongue and then progressively a broader front of the scraper edge engages the user's tongue as the scraper is drawn towards the tip of that tongue.

As can be seen in all of the embodiments of the present invention described above and depicted in the drawings typically the scraper includes a displacement kink between the far end of the handle and the distal end including the scraper edge of the arcuate head. In such circumstances, there is a natural tendency to present the arcuate head and so the scraper edge at an angle to a user's tongue to further improve sympathetic engagement with that tongue and through the bow or bite in the arcuate head to facilitate progressive engagement with the tongue as it is drawn across it in use.

It will be appreciated that removal of debris and bacteria from the rear or base of a user's tongue can be improved by incorporating in at least the distal end or scraper edge an anti-bacterial agent. This anti-bacterial agent can be provided as a component part of the material from which the distal end or scraper edge is formed or these portions of the scraper could incorporate means to allow them to be charged with anti-bacterial agent prior to use in cleaning a user's tongue. In addition to an anti-bacterial agent the arcuate head and in particular the distal end/scraper edge could include a breath freshener agent for distribution on the tongue to improve user breath.

A scraper in accordance with the present invention allows use of relatively cheap but typically previously unacceptable plastics materials in order to provide an acceptable scraper which can be readily disposable after an appropriate period of use. Thus, with a disposable scraper the necessity for repeated hygienic cleaning of the scraper is eliminated.

A scraper in accordance with the present invention can be made from a transparent, opaque or translucent plastic materials or polymers for aesthetic presentation in combination with coloured materials. Typically, transparent or opaque polymers are ABS, SAN (Styrene Acrylic Nitrile), co-polyesters, Acrylics and Polyester.

By providing a compliant scraper edge which is typically of an elastomeric form it will be appreciated that aggressive scraping of the tongue is reduced such that there is less reactive response from a user's tongue. Furthermore, the distinct difference between the compliant and typically elastomeric scraper edge and the remainder of the arcuate head provides a tactile difference to a user in order that the scraper is appropriately presented by that user by attempting to maintain contact substantially only through the scraper edge.

A further advantage of providing an arcuate head is that the hollow centre to that head can retain removed food and bacteria or debris removed from the user's tongue.

Where the arcuate head is detachable from the handle it will be appreciated that the handle can also be arranged to accommodate a supply of flossing tape for flossing between a user's teeth.

Whilst endeavouring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. A scraper for a human tongue, the scraper comprising an arcuate head with a distal end formed to compliantly engage upon a tongue.

2. A scraper as claimed as in claim 1 wherein the arcuate head is oval or round or an arch for structural stability to facilitate compliant engagement upon a tongue.

3. A scraper as claimed in claim 1 or claim 2 wherein the scraper includes a handle upon which the arcuate head is secured.

4. A scraper as claimed in any of claims 1,2 or 3 wherein the arcuate head is integral with the scraper.

5. A scraper as claimed in any of claims 1, 2 or 3 wherein the arcuate head is detachable from the scraper.

6. A scraper as claimed in any of claims 1, 2, 3 or 5 wherein the arcuate head is held in keyed engagement with the scraper.

7. A scraper as claimed in claim 6 or claim 7 wherein the arcuate head when attached to the scraper is locked in placed during use.

8. A scraper as claimed in any preceding claim wherein the distal end has a scraper edge for sympathetic engagement with a tongue.

9. A scraper as claimed in claim 8 wherein the scraper edge is made from a elastomeric material.

10. A scraper as claimed in any preceding claim wherein the distal end is serrated or otherwise shaped to facilitate scraping of a tongue.

11. A scraper as claimed in any preceding claim wherein the distal end, or when dependent upon claim 8 the scraper edge, is detachable from the arcuate head.

12. A scraper as claimed in any preceding claim wherein the distal end has a bow or bite for progressive engagement with a tongue as the scraper is drawn across a tongue in use.

13. A scraper as claimed in any preceding claim wherein the scraper if formed from plastics material which is conveniently cleaned.

14. A scraper as claimed in any preceding claim wherein the arcuate head is detachable for cleaning.

15. A scraper as claimed in any preceding claim wherein the distal end or the scraper edge includes an anti-bacterial agent or is charged with an anti-bacterial agent or breathe freshener.

16. A scraper as claimed in any preceding claim wherein at least the distal end or the scraper edge includes an agent to indicate either that the scraper should be cleaned or the anti-bacterial agent is exhausted or that the scraper should be replaced.

17. Any novel subject matter or combination including novel subject matter disclosed herein, whether or not within the scope of or relating to the same invention as any of the preceding claims.
